# EUROPÄISCHE PATENTSCHRIFT

(11) **EP 0 440 966 B1**
(45) Veröffentlichungstag und Bekanntmachung des Hinweises auf die Patenterteilung: **30.11.1994**
(21) Anmeldenummer: 90125136.3
(22) Anmeldetag: 21.12.1990
(51) Int. Cl.: A61K 7/48, A61K 7/40

(54) **Mittel zur Desinfektion der Haut und Verfahren zu seiner Herstellung**
Skin disinfectant composition and process for the production
Composition désinfectante pour la peau et procédé de préparation

(30) Priorität: 09.02.1990 DE 4004014
(43) Veröffentlichungstag der Anmeldung: 14.08.1991
(73) Patentinhaber: Felsch, Horst, Dr. Dipl.-Ing., A-6391 Fieberbrunn (AT)
(72) Erfinder: Felsch, Horst, Dr. Dipl.-Ing., A-6391 Fieberbrunn (AT)
(74) Vertreter: Patentanwälte Leinweber & Zimmermann

(56) Entgegenhaltungen:
- DE-A- 3 422 496
- GB-A- 2 155 337
- CHEMICAL ABSTRACTS, Band 80, Nr. 24, 17. Juni 1974, Seite 259, ZusammenfassungNr. 137174f, Columbus, Ohio, US; & SU-A-387 715 (V.V. CORBUNOVA et al.) 22-06-1973

## Beschreibung

Die Erfindung betrifft die Verwendung eines flüchtigen Rückfetters aus mindestens einem Methylcyclosiloxan der Formel (I)

[(CH₃)₂SiO]ₙ (I),

worin n eine ganze Zahl von 3 bis 9 darstellt, in einem Mittel zur Desinfektion der Haut, insbesondere der Hände, auf der Basis eines Alkohol/Wasser-Gemisches.

Ein solches Desinfektionsmittel soll hautverträglicher sein bei gleichzeitig besserer Desinfektionswirkung als die bisher bekannten alkoholischen Haut- und Händedesinfektionsmittel.

Auf vielen Gebieten des täglichen Lebens, insbesondere in der Medizin, ist es häufig erforderlich, Haut und Hände zu desinfizieren, das heißt, in einen Zustand zu bringen, in dem sie nicht mehr infizierend wirken. Da es nicht möglich ist, die Haut völlig keimfrei zu machen, bleiben auch auf der desinfizierten Haut immer noch einige Keime zurück. Um eine Infektion zu verhüten, ist es wesentlich, eine möglichst starke Reduktion der ursprünglichen Keimzahl zu erreichen. Mit den wenigen verbleibenden Keimen kommt das Abwehrsystem des Körpers dann zurecht, so daß letztlich keine Infektion stattfindet.

Bei der Desinfektion der Hände unterscheidet man zwei verschiedene Arten, nämlich
- die hygienische Händedesinfektion und
- die chirurgische Händedesinfektion.

Bei der hygienischen Händedesinfektion sollen die sogenannten Anflugkeime als potentielle Infektionserreger möglichst noch vor einem Waschvorgang abgetötet werden, wobei es hier vor allein auf eine äußerst rasch eintretende Wirkung ankommt. Der Anwender soll nicht warten müssen. Eine ausreichende Keimreduktion muß schon nach 30 Sekunden bis spätestens einer Minute Anwendung des Desinfektionsmittels erbracht werden (Sofortwirkung).

Bei der chirurgischen Händedesinfektion ist es wichtig, daß der Chirurg auch bei einer längeren Operation sicher sein kann, daß trotz eines vielleicht nicht ganz dichten Operationshandschuhs der sich darunter gebildete Handschuhsaft nicht infizierend wirkt. Bei der chirurgischen Händedesinfektion kommt es demnach auf eine Langzeitwirkung an Der Chirurg desinfiziert Hände und Unterarme, zieht danach den sterilen Operationshandschuh über und darf nun bei Anwendung eines geeigneten chirurgischen Händedesinfektionsmittels die Sicherheit haben, daß der Patient innerhalb von 3 Stunden durch seine Hände - auch wenn der Handschuh durch einen Schnitt verletzt sein sollte - nicht infiziert wird (Langzeitwirkung bzw. remanente Wirkung).

Als Desinfektionsmittel werden meist hochprozentige Gemische niedriger Alkohole verwendet, da die gewünschten hohen und raschen Keimreduktionen nur durch alkoholische Präparate erbracht werden. Dafür stehen vor allem Ethylalkohol, Isopropanol und n-Propanol zur Verfügung. Methanol wird aufgrund seiner Giftigkeit ausgeschlossen, während Butanole wegen ihres unangenehmen Eigengeruches ebenfalls nicht eingesetzt werden. Die keimtötende Wirkung nimmt zu in der Reihenfolge Ethanol, i-Propanol und n-Propanol, wobei gilt, daß 80 vol.-%-iges Ethanol etwa gleich wirksam ist wie 60 %-iges Isopropanol, das wiederum in seiner Desinfektionswirkung mit 40 vol.-%-igem n-Propanol vergleichbar ist. Moderne hygienische Desinfektionsmittel bestehen daher heute meistens aus einem Gemisch von Isopropanol und n-Propanol. Diese beiden Komponenten werden auch als Desinfektionsstandards verwendet.

So muß beispielsweise ein Mittel zur hygienischen Händedesinfektion zumindest die gleiche Wirkung zeigen wie ein 60 vol.-%-iges Isopropanol innerhalb einer Einwirkungszeit von 30 Sekunden bis maximal 1 Minute, während bei der chirurgischen Händedesinfektion als Standard 60 vol.-%-iges n-Propanol bei einer Einwirkungszeit von bis zu 5 Minuten (die Wirkung wird nach 3 Stunden gemessen) verwendet wird. Die desinfizierende Wirkung wird in Form von Reduktionsfaktoren angegeben. Man verstehet darunter die Differenz zwichen dem Logarithmus der Ausgangskeimzahl und dem Logarithmus der Keimzahl nach der Desinfektion (logarithmischer Reduktionsfaktor). Die Desinfektionswirkung von Händedesinfektionsmitteln wird beispielsweise nach den Richtlinien für die Prüfung und Bewertung chemischer Desinfektionsverfahren erster Teilabschnitt, 1.1.1981, in der jeweils aktuellen Fassung bestimmt.

Bei einem Gehalt von 80 Vol.-% wird das Wirkungsmaximum erreicht. Eine weitere Erhöhung der Alkoholkonzentration fuhrt zu einer Abnahme der Desinfektionswirkung, wobei wasserfreie Alkohole nahezu unwirksam sind.

Die heute gebräuchlichen Mittel zur Desinfektion der Haut, insbesondere der Hände, die hochprozentige Alkoholgemische der vorgenannten Art darstellen haben jedoch den Nachteil, daß sie schlecht hautverträglich sind. Dies hat seine Ursache darin, daß Alkohole sehr hygroskopisch sind und der Haut Feuchtigkeit entziehen. Sie machen sie rissig und spröde. Dies wiederum fordert die Infektionsgefahr. Da andererseits alkoholische Desinfektionsmittel vor allem solche für die rasche, hygienische Händedesinfektion, sehr oft angewendet werden, beispielsweise bis zu 80 mal am Tage bei Zahnärzten, wäre gerade für diese Produkte eine möglichst gute Hautverträglichkeit sehr erwünscht.

Dazu kommt, daß hochprozentige Alkoholgemische das auf der natürlichen Haut befindliche Fett herauslösen. Nach der Verdunstung der Alkohole wird dieses oberflächlich zurückgelassen. Die Haut sieht dadurch an manchen Stellen weiß gefleckt aus.

Diese Nachteile, ein unerwünschter Wasserentzug und eine unerwünschte Entfettung, Wurden bisher Symptomatisch dadurch bekämpft, daß den Desinfektionsmitteln sogenannte Rückfetter zugesetzt wurden, die pflegend auf die Haut wirken, eine Sprödigkeit verhindern und das abgelagerte Hautfett nicht sichtbar erscheinen lassen. Beispiele für solche Rückfetter sind
- höhere Alkohole, wie Glycerin, Propylenglycol, Diethylenglycol ;
- Öle und Fette, wie Rhizinusöl, Olivenöl, gesättigte Triglyceride z. B. Glyceride mit einer Kohlenstofflänge von 8 bis 12 Kohlenstoffatomen ;
- Ester, wie Isopropylmyristat, Polyolfettsäureester; und
- Äther, wie Adipinsäurediisopropyläther.

Diese Rückfetter-Zusätze vermitteln zwar dem Anwender zunächst ein gutes Hautgefühl ohne Weitere Spannungszustände, auf die Dauer treten aber die folgenden Nachteile auf:
- die herkömmlichen Rückfetter haben bei der Oberflächentemperatur der Haut (bei der Handfläche sind dies bis zu 35°C) einen sehr geringen Dampfdruck und verflüchtigen sich dadurch nicht. Bei jedem Auftrag von mindestens 3 ml Desinfektionsmittel auf beide Handflächen verdunsten also die Alkohole und das enthaltene Wasser sehr rasch. Zurück bleiben die Rückfetter, die sich mehr und mehr auf der Hautoberfläche aufkonzentrieren.
   Bei einer Anwendung bis zu 80 mal am Tag, wie dies z.B. bei Zahnärzten der Fall ist, kommt es daher zu massiven Hautüberfettungen.
   Diese Überfettung führt oftmals zu einem Gegenteil des beabsichtigten Effektes. Die Hautverträglichkeit sinkt wieder ab:
- Durch die auf der Haut zurückbleibenden, massiven Rückfetteranteile wird der keimtötende Angriff des Alkohols Verzögert und erschwert. Der Alkohol muß ja zunächst durch eine Rückfetterschicht hindurch bis zu den Mikroorganismen vordringen. Er kann erst dann wirksam werden, wenn er durch die Zellwand eintritt und im Zellinneren Eiweiß denaturiert. "Eingefettete" Mikroorganismen sind schwerer abzutöten.
- Überfettete Hände sind vor allem für jene Anwender unangenehm, die ein besonderes Fingerspitzengefühl für ihre Arbeit benötigen. Der Zahnarzt kann mit überfetteten Händen genauso wenig arbeiten, wie der praktische Arzt beim Setzen einer punktgenauen Injektion.
   Auch für das medizinische Verwaltungspersonal hat sich die Anhäufung von Rückfettern besonders nachteilig ausgewirkt; die Karteikarten haben im Laufe der Zeit Fettflecke erhalten und sind dadurch unansehnlich geworden.
- Paraffinöle und Glycerin werden durch die Haut resorbiert. Bei Glycerin beträgt die Resorptionsgeschwindigkeit 0,2 mg/cm² Haut und Stunde bei 25°C. Bei einem durchschnittlichen Menschen wird die Hautoberfläche mit 1,73 m² angenommen. Ein Prozent davon bildet eine Handfläche. Beide Handflächen (das ist die eigentliche Handfläche und der Handrücken beider Hände) haben zusammen 346 cm². Pro Stunde können somit von beiden Handflächen knapp 70 mg Glycerin resorbiert werden. Dazu kommt der Nachteil , daß Glycerin selbst ein Alkohol und damit auch stark hygroskopisch ist.
   Auch bei Paraffinöl ist eine massive Hautresorption bekannt. Das resorbierte Paraffinöl wird dabei oftmals in Vakuolen eingekapselt und im Körper als Lipone abgelagert.
- Es ist bekannt, daß lipophile, also fettähnliche Rückfetter, Vermittler für eine beschleunigte Hautresorption von Desinfektionswirkstoffen (z.B. Chlorhexidindiglukonat, Phenylquecksilberborat, verschiedene substituierte Phenole) sein können. Diese Langzeit-Desinfektionswirkstoffe sind daher oftmals im Harn des Anwenders nachweisbar.
- Die Hautunverträglichkelt, die speziell von derartigen chirurgischen Desinfektionsmitteln ausgeht, hatihre Ursache darin, daß diese die natürliche Schutzflora der Haut über längere Zeit abtöten und damit ihre Regenerationsfähigkeit behindern. Die Schutzflora der Haut ist wesentlich, um den natürlichen Säuremantel wieder aufzubauen und damit einen Selbstschutz gegen Anflugkeime, z.B. E. coli, zu entwickeln. Ohne natürliche Schutzflora gibt es auch keine Entwicklung des Säuremantels. Derartige langzeitwirksame Hautdesinfektionsmittel müssen daher a priori hautunverträglich sein.

Ein praktisches Beispiel: Ein Händedesinfektionsmittel hat nach dem derzeitigen Stand der Technik folgende Zusammensetzung:
70 Vol.-% eines Gemisches, bestehend aus Iso- und n-Propanol,
28 Vol-% Wasser,
2 Vol.-% Isopropylmyristat als Rückfetter.

Isopropylmyristat wird in großem Maße in der Kosmetik angewendet. Es ist ein hautpflegender Zusatzstoff, der gut in die Haut eindringt.
Mit 3 ml dieses Desinfektionsmittels gelangen nun auf beide Handflächen: 2,1 ml iso- und n-Propanol, 0,84 ml Wasser und 0,06 ml Isopropylmyristat. Die Alkohole dringen also einerseits über die Zellmembranen in die abzutötenden Mikroorganismen ein und bewirken dort eine Eiweißfällung. Andererseits werden durch diese Alkohole auch die Hautfette teilweise gelöst. Hautfette bestehen vor allem aus freien und veresterten Fettsäuren, sind reich an Neutralfetten, enthalten zusätzlich Wachse und Glycerin sowie unverseifbare Fette in Form von Cholesterin und ungesättigte Alkoholen neben einem relativ hohen Anteil an Lanolin.

Obwohl wäßrige Alkohole ansonsten kaum Triglyceride lösen können, ist die Löslichkeit in diesem genannten Hautfettgemisch doch bedeutend. Das Fettgemisch wird also herausgelöst und in die flüssige Phase des Desinfektionsmittels transportiert. Beschleunigt durch die Hautoberflächentemperatur der Hände (maximal 35°C) beginnt gleichzeitig die rasche Verdunstung der Alkohole. Die Verdunstungsgeschwindigkeit kann hier über die Verdunstungszahl angegeben Werden.

[Definition der Verdunstungszahl: Das ist jene Zeit in Sekunden, in der 0,2 ml Substanz bei 50 % relativer Luftfeuchtigkeit und 23°C bei stehender Luft nach Augenschein getrocknet sind. Diethyläther mit einen Siedepunkt von etwa 35°C bekommt hierbei die Zahl 1, Isopropanol hat die Verdunstungszahl 21. Das heißt, Isopropanol benötigt 21 mal länger, um bei den genannten Bedingungen zu verdunsten als Diethyläther].

Durch diese rasche Verdunstung werden die im Alkoholgemisch gelösten Hautfette wieder ausgeschieden. Zurück bleibt eine Wasser-Isopropylmyristat-Emulsion. Ein Teil des von den Alkoholen wieder freigesetzten Hautfettes wird nun durch das Isopropylmyristat gelöst. Die Emulsion mit dem Wasser behindert diesen Vorgang etwas. Isopropylmysistat hat eine Verdunstungszahl von etwa 4 350. Damit ist diese Substanz um den Faktor 200 langsamer verdunstbar als das Alkoholgemisch. Die zugesetzten 0.06 ml Isopropylmyristat verbleiben mit dem darin gelösten Hautfett auf der Handoberflache und lagern sich vor allem in den Poren ab.

Nach etwa 5 Anwendungen sind bereits 0,3 ml Isopropylmyristat auf etwa 346 cm² Handoberfläche verteilt. Damit ist das Porenvolumen endgültig gefüllt. Ein jeder weitere Zusatz dieses Rückfetters bedeutet bereits Überfettung und fettigen Griff. Gleichzeitig beginnt die Hautresorption und damit auch die verstärkte Aufnahme der im Isopropylmyristat gelösten, langzeitwirksamen Desinfektionsmittel (z. B. substituierte Phenole).

Es ist typisch für die derzeit eingesetzten Rückfetter, daß sie nicht nur das vom Alkohol gelöste Hautfettgemisch aufnehmen, sondern daß sie es durch ihre eigene Anwesenheit vermehren und in der Zusammensetzung verändern.

In der Firmenschrift von DOW CORNING: "Information about volatile Silicone Fluids" (1982) sind Methylcyciosiloxane beschrieben und hierfür eine ganze Reihe von Eigenschften angegeben, u. a. auch die Eigenschaft 'nongreasy'. Über eine Rückfettwirkung dieser Silikone ist jedoch nichts ausgesant oder dies nur angedeutet, ganz im Gegenteil ergibt sich aus der Charakterisierung 'nongreasy', daß ein Fachmann auf dem betreffenden Gebiet wohl kaum daran gedacht hätte, sondern gerade davon angehalten worden wäre, solche Methylcyclosiloxane als Rückfetter in einem Desinfektionsmittel einzusetzen.

Aus G. A. Nowak "Die kosmetischen Präparate", 2. AufLage, (1984), Seite 139 und Seiten 391 bis 395, ergeben sich eine Reihe von Angaben über die Zusammensetzung von kosmetischen Präparaten, die Verwendung von Methylcyclosiloxanen ist hierin jedoch nicht angesprochen.

Aufgabe der Erfindung war es daher, die bekannten alkoholischen Desinfektionsmittel so weiterzuentwickeln, daß sie die vorstehend geschilderten Nachteile nicht mehr besitzen, insbesondere eine verbesserte Hautverträglichkeit aufweisen.

Diese Aufgabe wird erfindungsgemäß dadurch gelöst, daß in dem alkoholischen Desinfektionsmittel auf Basis eines Alkhol/Wasser-Gemisches, anstelle des bisher eingesetzten Rückfetters ein flüchtiger Rückfetter aus mindestens einem Methylcyclosiloxan der allgemeinen Formel (I) verwendet wird:

[(CH₃)₂SiO]ₙ (I)

worin n eine Zahl von 3 bis 9, vorzugsweise von 3 bis 5, insbesondere die Zahl 4, darstellt.

Die erfindungsgemäß verwendeten neuartigen Rückfetter haben bei 35°C, der durchschnittlichen Hautoberflächentemperatur der Hand, einen deutlich meßbaren Dampfdruck, so daß diese schon nach einer kurzen Verweilzeit auf der Haut langsam in die Gasphase übergehen und rückstandslos verdampfen.

Als Rückfetter in dem Desinfektionsmittel besonders gut geeignet ist das Octamethylcyclotetrasiloxan der nachstehenden Formel 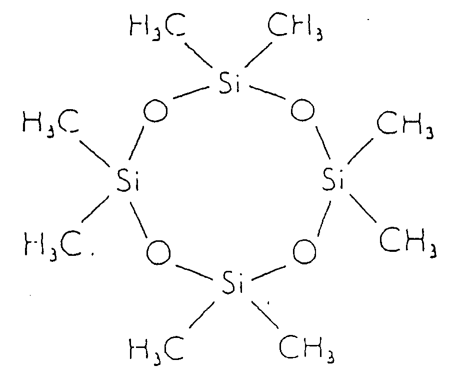
Die relative Verdunstungszahl dieser Verbindung (gemäß der weiter oben angegebenen Definition) beträgt 70. (Zum Vergleich: Wasser 42, Isopropanol 21, Glycerin 2625 und Iso-propylmyristat 4350). Der Dampfdruck in absoluten Zahlen beträgt bei 20°C etwa 10 mbar, bei 35°C etwa 25 mbar. Im Vergleich dazu hat das Hexamethylcyclolrisiloxan bei einem Siedepunkt von 133°C (bei Atmosphärendruck) bei 35°C einen Dampfdruck von etwa 160 mbar - es verflüchtigt sich demnach auf der Hautoberfläche noch schneller als das Octamethylcyclotetrasiloxan. Erfindungsgemäß besonders gut geeignet ist auch das Decamethylcyclopentasiloxan, das bei einer Siedetemperatur von etwa 210°C (bei Atmosphärendruck) einen Dampfdruck bei 20°C von etwa 1 mbar und bei 35°C von etwa 5 mbar hat. Seine relative Verdunstungszahl beträgt 360 und ist damit immer noch deutlich besser als diejenige von Glycerin mit 2625 bzw. von Isopropylmyristat mit 4350.

Durch das Mischen von Hexamethylcyclotrisiloxan, Octamethylcyclotetrasiloxan und Decamethylcyclopentasiloxan können die erfindungsgemäß verwendeten Rückfetter genau auf den speziellen Anwendungszweck eingestellt werden. Der Zahnarzt oder praktische Arzt, der seine Hände sehr häufig desinfizieren muß, wird dafür in der Regel einen höherflüchtigen Rückfetter verwenden als der Chirurg, der nach einmaliger Händedesinfektion etwa 3 Stunden arbeitet. Höherflüchtige Rückfetter enthalten mehr Hexamethylcyclotrisiloxan und weniger Decamethylcyclopentasiloxan.

In dem alkoholischen Desinfektionsmittel ist der erfindungsgemäß verwendete flüchtige Methylcyclosiloxan-Rückfetter der Formel (I) vorzugsweise in einer Menge von 0,1 bis 12 Vol.-%, insbesondere in einer Menge von 2 bis 6 Vol.-%, jeweils bezogen auf die Gesamtmischung, enthalten.

Das alkoholische Desinfektionsmittel enthält als Alkohol vorzugsweise Ethylalkohol, Isopropanol und/oder n-Propanol in einer Gesamtmenge von bis zu 80 Vol.-% (der Rest ist Wasser), insbesondere in einer Gesamtmenge von 40 bis 80 Vol.-%, jeweils bezogen auf das Gesamtgemisch.

Besonders bevorzugt ist die Verwendung eines Gemisches aus i-Propanol und n-Propanol als Alkoholkomponente des Desinfektionsmittels.

Zur Verbesserung der Hautverträglichkeit werden überdies zweckmäßig Pufferlösungen zugesetzt, mit deren Hilfe eine Einstellung auf den natürlichen pH Wert der Haut (5,5) möglich ist. Hierfür ist besonders geeignet Ammonlactat, weil dieses zusätzlich aufgrund seiner hygroskopischen Eigenschaft die desinfizierte Haut mit Feuchtigkeit versorgt.

Das Desinfektionsmittel liegt im allgemeinen in Form einer Lösung vor, die nach einem einen weiteren Gegenstand der Erfindung bildenden Verfahren dadurch erhalten wird, daß der flüchtige Methylcyclosiloxan-Rückfetter der oben angegebenen allgemeinen Formel (I) in der Alkohol-Basis gelöst und dann mit Wasser, vorzugsweise destilliertem Wasser, auf die gewünschte Menge auf gefüllt wird unter Bildung einer Desinfektionslösung.

Ein besonders vorteilhaftes alkoholisches Desinfektionsmittel enthält als flüchtigen Rückfetter ein Gemisch aus 85 Vol.-% Octamethylcyclotetrasiloxan und 15 Vol.-% Decamethylcyclopentasiloxan.

Da die erfindungsgemäß verwendeten flüchtigen Silikone in Wasser unlöslich sind, müssen sie bei der Herstellung alkoholischer Haut- und Händedesinfektionsmittel zuerst in Alkohol gelöst werden, wobei bis zu 80 Vol.-% Alkohol eingesetzt werden können. Da diese Desinfektionsmittel ohne Wasser jedoch nicht wirksam sind (ein Wasserzusatz von 20 Vol.-% stellt ein Wirkunsoptimum dar), muß anschließend Wasser, vorzugsweise destilliertes Wasser, der so erhaltenen Lösung zugesetzt werden. Auf diese Weise ist es möglich, dem Alkohol/Wasser-Gemisch bis zu 12 Vol.-% flüchtige Methylcyclosiloxane der allgemeinen Formel (I) zuzusetzen, ohne daß Entmischungen auftreten. In der Regel genügt ein zusatz von 2,5 Vol.-%, wobei für bestimmte Zwecke aber auch schon Zusätze von nur 0,1 Vol.-% ausreichend sein können.

Obgleich der genaue Wirkunsmechanismus der im Desinfektionsmittel enthaltenen Rückfetters noch nicht völlig geklärt ist, wird angenommen, daß ihre Wirkung darin besteht, daß das von den Alkoholen herausgelöste Hautfett in erfindungsgemäß verwendeten Methylcyclosiloxan-Rückfetter gelöst wird. Durch ihren ausgeprägten Spriting-Effekt und die bei der Desinfektion durchzuführenden Handbewegungen wird die dabei erhaltene Fettlösung auf der gesamten Oberfläche der desinfizierten Haut verteilt, wobei das entzogene Fett somit der Haut wieder zurückgegeben wird, wenn anschließend der MethylcyclosiloxanRückfetter rückstandslos verdampft. Die Vorteile des Mittels zur Desinfekion der Haut, insbesondere der Hände, auf der Basis eines Alkohol/Wasser-Gemisches gegenüber den bekannten alkoholischen Desinfektionsmitteln sind insbesondere folgende:
- die Verwendung flüchtiger Silikone der oben angegebenen Formel (I) verhindert die Anhäufung von Rückfettern auf der Haut, die bei den bisher verwendeten nicht-flüchtigen Rückfettern eine Hautunverträglichkeit durch Überfettung, einen Verlust an dem für bestimmte Berufsgruppen (z.B. Zahnärzte und Ärzte) besonders wichtigen Hautgriff, die Entstehung von Fettflecken auf Karteikarten und den Drang, die Hände häufiger als nötig zu waschen, hervorrief;
- die erfindungsgemäß als Rückfetter verwendeten flüchtigen Silikone verleihen dem Desinfektionsmittel eine hervorragende Hautverträglichkeit;
- als Folge der Flüchtigkeit des erfindungsgemäß verwendeten Rückfetters bleibt auf der Haut kein Vehikel zurück, das beispielsweise die Resorption von nicht-flüchtigen Desinfektionswirkstoffen durch die Haut fördern könnte. Dadurch wird auch die Schutzflora der Haut weniger stark angegriffen, was ebenfalls zu einer besseren Hautverträglichkeit führt;
- nach dem Auftrag des Desinfektionsmittels auf die Haut verflüchtigt sich zunächst der darin enthaltene Alkohol. Die von diesem gelösten Hautfette werden dann von den erfindungsgemäß verwendeten flüchtigen Silikonölen aufgenommen und durch das hohe Spriting-Vermögen und die Handbewegungen beim Desinfizieren wieder vollständig auf die desinfizierte Hautoberfläche verteilt. Danach verdampft das flüchtige Silikonöl rückstandslos. Das heißt, das vom Alkohol gelöste Hautfett wird von den flüchtigen Silikonölen der Haut wieder in gleichmäßiger Verteilung zugeführt, wobei es zu keiner Vermischung von Hautfett und Rückfetter-Fett kommt, so daß die natürliche Zusammensetzung des Hautfettes keine Veränderung erfährt.

Aus den obengenannten Gründen weist das Desinfektionsmittel eine verbesserte Hautverträglichkeit auf, so daß auch hochprozentige alkoholische Desinfektionsmittel sehr häufig am Tage angewendet werden können. Da das Mittel zur Desinfektion der Haut, insbesondere der Hände, nach dem Verdunsten seiner Alkohol- und Siloxan-Bestandteile keine Rückstände hinterläßt, sondern der Haut lediglich das vorher entzogene Fett wieder zuführt, wobei der Verbrauch an Verdunstungswärme nur gering ist, entsteht kein kühles Hautgefühl, die Haut erhält samtweiche Eigenschaften und es entsteht kein öliges Gefühl. Das Desinfektionsmittel ist auch gut verträglich mit Kosmetika, wie sie üblicherweise auf die Haut aufgetragen werden. Es ruft auch keinerlei Hautirritationen hervor. Der erfindungsgemäße Rückfetter hat keinen lösungsmitteltypischen Geruch und bewirkt, daß Duftstoffe die im erfindungsgemäß verwendeten Hautdesinfektionsmittel enthalten sind, auf der Haut länger haften als bei Anwendung der bekannten Hautdesinfeksionsmittel. Es ist auch umweltfreundlich, da es vollständig verdampft, so daß kein Rückfetter in das Abwassersystem gelangt.

Die Erfindung wird durch die folgenden Beispiele näher erläutert, ohne jedoch darauf beschränkt zu sein.

### Beispiel 1

Zur Herstellung von 1 l eines Haut- und Händedesinfektionsmittels mit flüchtigem Rückfetter werden eingesetzt (Standardpräparation):
700 ml Isopropanol
25 ml Octamethylcyclotetrasiloxan
ad 1000 ml mit destilliertem Wasser aufgefüllt.

(Die theoretische Menge von 275 -l würde nicht genau stimmen, da Alkohol und Wasser miteinander vermischt eine Volumenkontraktion ergeben, daher die Formulierung "ad 1000 ml mit destilliertem Wasser aufgefüllt".)

### Beispiel 2

Zur Herstellung von 1 l eines Haut- und Händedesinfektionsmittels mit flüchtigem Rückfetter werden eingesetzt (bis 5 Auftragungen pro Tag):
500 ml n-Propanol
300 ml Isopropanol
25 ml Decamethylcyclopentasiloxan
ad 1000 ml mit destilliertem Wasser aufgefüllt.

### Beispiel 3

Zur Herstellung von 1 l eines Haut- und Händedesinfektionsmittels mit flüchtigem Rückfetter werden eingesetzt (bis 10 Auftragungen pro Tag):
500 ml n-Propanol
300 ml Isopropanol
30 ml Octamethylcyclotetrasiloxan
ad 1000 ml mit destilliertem Wasser aufgefüllt.

### Beispiel 4

Zur Herstellung von 1 l eines Haut- und Händedesinfektionsmittels mit flüchtigem Rückfetter werden eingesetzt (bis 20 Auftragungen pro Tag):
500 ml n-Propanol
300 ml Isopropanol
12 ml Octamethylcyclotetrasiloxan
7 ml Decamethylcyclopentasiloxan
6 ml Hexamethylcyclotrisiloxan
ad 1000 ml mit destilliertem Wasser aufgefüllt.

### Beispiel 5

Zur Herstellung von 1 l eines Haut- und Händedesinfektionsmittels mit flüchtigem Rückfetter werden eingesetzt (für besonders trockene Hände und bis 20 Auftragungen pro Tag):
600 ml n-Propanol
100 ml Isopropanol
50 ml Octamethylcyclotetrasiloxan
ad 1000 ml mit destilliertem Wasser aufgefüllt.

### Beispiel 6

Zur Herstellung von 1 l eines Haut- und Händedesinfektionsmittels mit flücht-igem Rückfetter werden eingesetzt (für mehr als 50 Auftragungen pro Tag):
500 ml n-Propanol
250 ml Isopropanol
40 ml Hexamethylcyclotrisiloxan
20 ml Octamethylcyclotetrasiloxan
ad 1000 ml mit destilliertem Wasser aufgefüllt.

## Patentansprüche

1. Verwendung eines flüchtigen Rückfetters aus mindestens einem Methylcyclosiloxan der allgemeinen Formel (I)
[(CH₃)₂SiO]ₙ (I)
worin n eine Zahl von 3 bis 9 darstellt,
in einem Mittel zur Desinfektion der Haut, insbesondere der Hände, auf der Basis eines Alkohol/Wasser-Gemisches.

2. Verwendung nach Anspruch 1, dadurch gekennzeichnet, daß ein Methylcyclosiloxan der Formel (I) verwendet wird, worin n für eine Zahl von 3 bis 5 steht.

3. Verwendung flach Anspruch 1 oder 2, dadurch gekennzeichnet, daß als Methylcyclosiloxan der Formel (I) Octamethylcyclotetrasiloxan der Formel 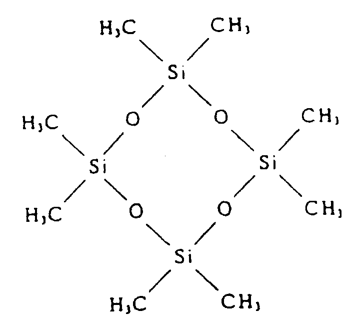 verwendet wird.

4. Verwendung nach einem der Ansprüche 1 bis 3, dadurch gekennzeichnet, daß das Methylcyclosiloxan der Formel (I) in einer Menge von 0,1 bis 12 Vol.-% verwendet wird.

5. Verwendung nach Anspruch 4, dadurch gekennzeichnet, daß das Methylcyclosiloxan der Formel (I) in einer Menge von 2 bis 6 Vol.-% verwendet wird.

6. Verwendung nach einem der Ansprüche 1 bis 5 in einem Gemisch, das als Alkohol Ethylalkohol, Isopropanol und/oder n-Propanol in einer Gesamtmenge von bis zu 80 Vol.%, bezogen auf das Gesamtgemisch, enthält.

7. Verwendung nach Anspruch 6 in einem Gemisch, das als Alkohol ein Gemisch aus Isopropanol und n-Propanol enthält.

8. Verwendung nach einem der Ansprüche 1 bis 7 in einem Gemisch, das Pufferlösungen aus Phosphaten, Lactaten, und/oder Acetaten, insbesondere unter Berücksichtigung von deren Ammonsalzen, wie Ammonlacaten oder Ammonacetat, enthält.

9. Verwendung nach einem der Ansprüche 1 bis 8 in einem Gemisch, in dem das Methylcyclosiloxan in gelöster Form vorliegt.

## Claims

1. Use of a volatile superfatting agent composed of at least one methylcyclosiloxane of the general formula (I)
[(CH₃)₂SiO]ₙ (I)
in which n represents a number from 3 to 9, in an agent for the disinfection of the skin, especially of the hands, based on an alcohol/water mixture.

2. Use according to Claim 1, characterized in that a methylcyclosiloxane of the formula (I) in which n represents a number from 3 to 5 is used.

3. Use according to Claim 1 or 2, characterized in that octamethylcyclotetrasiloxane of the formula 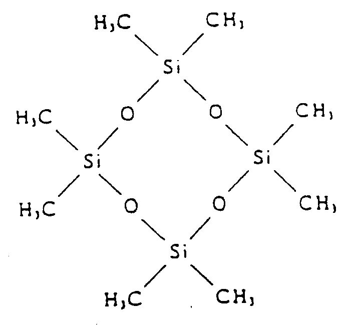 is used as methylcyclosiloxane of the formula (I).

4. Use according to any of Claims 1 to 3, characterized in that the methylcyclosiloxane of the formula (I) is used in an amount of from 0.1 to 12 % by volume.

5. Use according to Claim 4, characterized in that the methylcyclosiloxane of the formula (I) is used in an amount of from 2 to 6 % by volume.

6. Use according to any of Claims 1 to 5, in a mixture which contains as alcohol ethyl alcohol, isopropanol and/or n-propanol in a total amount of up to 80 % by volume, based on the complete mixture.

7. Use according to Claim 6 in a mixture which contains as alcohol a mixture of isopropanol and n-propanol.

8. Use according to any of Claims 1 to 7, in a mixture which contains buffer solutions composed of phosphates, lactates and/or acetates, with particular regard for the ammonium salts thereof, such as ammonium lactates or ammonium acetate.

9. Use according to any of Claims 1 to 8, in a mixture in which the methylcyclosiloxane is present in dissolved form.

## Revendications

1. Utilisation d'un agent regraissant volatil formé d'au moins un méthylcyclosiloxane ayant la Formule générale (I) :
[(CH3)2SiO]n (I)
où n est un nombre compris entre 3 et 9, dans un agent pour La désinfection de la peau, en particulier des mains, à base d'un mélange alcool/eau.

2. Utilisation selon la revendication 1, caractérisée en ce qu'on utilise un méthylcyclosiloxane de la Formule (I), où n est un nombre compris entre 3 et 5.

3. Utilisation selon la revendication 1 ou 2, caractérisée en ce qu'on utilise, en tant que méthylcyclosiloxane de la Formule (I), de l'octaméthylcyclosiloxane de la Formule

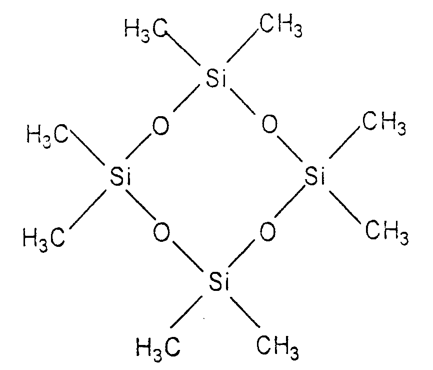

4. Utilisation selon l'une quelconque des revendications 1 à 3, caractérisée en ce que le méthylcyclosiloxane de la Formule (I) est utilisé en une quantité de 0,1 à 12% en volume.

5. Utilisation selon la revendication 4, caractérisée en ce que le méthylcyclosiloxane de la Formule (I) est utilisé en une quantité de 2 à 6% en volume.

6. Utilisation selon l'une des revendications 1 à 5, dans un mélange qui contient, en tant qu'alcool, de l'alcool éthylique, de l'isopropanol et/ou du n-propanol en une quantité totale pouvant atteindre 80% en volume, en se rapportant au mélange total.

7. Utilisation selon la revendication 6, dans un mélange qui contient, en tant qu'alcool, un mélange d'isopropanol et de n-propanol.

8. Utilisation selon l'une des revendications 1 à 7, dans un mélange qui contient des solutions tampons formées de phosphates, lactates et/ou acétates, en particulier en considérant leurs sels d'ammonium, comme les lactates d'ammonium ou l'acétate d'ammonium.

9. Utilisation selon l'une des revendications 1 à 8, dans un mélange dans lequel le méthylcyclosiloxane est présent sous forme dissoute.
